# EUROPÄISCHE PATENTSCHRIFT

(11) **EP 2 855 647 B1**
(45) Veröffentlichungstag und Bekanntmachung des Hinweises auf die Patenterteilung: **10.08.2016**
(21) Anmeldenummer: 13726756.3
(22) Anmeldetag: 29.05.2013
(51) Int. Cl.: A61Q 19/00, A61K 8/34, A61K 8/41, A61Q 5/12, A61K 8/06, A61K 8/42

(54) **ZUSAMMENSETZUNGEN ENTHALTEND FETTALKOHOLE, KATIONISCHE TENSIDE UND N-ACYL-N-METHYLGLUCAMINE**
COMPOSITIONS CONTAINING FATTY ALCOHOLS, CATIONIC SURFACTANTS AND N-ACYL-N-METHYLGLUCAMINES
COMPOSITIONS CONTENANT DES ALCOOLS GRAS, DES TENSIOACTIFS CATIONIQUES ET DES N-ACYL-N-MÉTHYLGLUCAMINES

(30) Priorität: 30.05.2012 DE 102012010699; 20.04.2013 DE 102013006878
(43) Veröffentlichungstag der Anmeldung: 08.04.2015
(73) Patentinhaber: CLARIANT INTERNATIONAL LTD, 4132 Muttenz (CH)
(72) Erfinder: KLUG, Peter, 63762 Großostheim (DE); DAHMS, Gerd, 47138 Duisburg (DE); MILDNER, Carina, 65931 Frankfurt am Main (DE)
(74) Vertreter: Holmes, Rosalind
(86) Internationale Anmeldenummer: PCT/EP2013/061106
(87) Internationale Veröffentlichungsnummer: WO 2013/178701

(56) Entgegenhaltungen:
- EP-A1- 0 285 768
- WO-A1-94/21226
- WO-A1-95/19415
- WO-A1-97/47284

## Beschreibung

Die Erfindung betrifft Zusammensetzungen, bevorzugt kosmetische, dermatologische und pharmazeutische Zusammensetzungen, enthaltend Fettalkohole, kationische Tenside und bestimmte N-Acyl-N-Methylglucamine, deren Verwendung zur Pflege der Haut und der Haare sowie die Verwendung der N-Acyl-N-Methylglucamine zur Verdickung und Verbesserung der Pflegewirkung kationischer Emulsionen.

Pflegemittel zur Pflege der Haut und der Haare, enthaltend N-Acyl-N-Methylglucamine, Fettalkohole und quaternäre Ammoniumverbindungen, die mindestens eine langkettige Alkyl- oder Alkenylgruppe aufweisen, beispielsweise Behenyltrimethylammoniumchlorid, Stearyltrimethylammoniumchlorid, Lauryltrimethylammoniumchlorid, Cetyltrimethylammoniumchlorid, Distearyldimethylammoniumchlorid, sind bekannt.

Weiterhin bekannt sind kosmetische Zusammensetzungen enthaltend Fettsäure-N-alkylpolyhydroxyalkylamide und Fettalkohole.

In der WO 97/47284 werden neue kosmetische Zubereitungen für die Anwendung im Bereich der Haar- und Hautpflege vorgeschlagen enthaltend (a) Esterquats und (b1) Sorbitanester, (b2) Polyolpoly-12-hydroxystearate und/oder (b3) Glyceride sowie gegebenenfalls (c1) Alkyl- und/oder Alkenyloligoglykoside und/oder (c2) Fettsäure-N-alkylpolyhydroxyalkylamide. Die Mittel zeichnen sich durch einen verbesserten Weichgriff der Haare und ein besonders angenehmes Hautgefühl aus. Konkret offenbart sind Beispiele mit Fettsäure-N-alkylpolyhydroxyalkylamiden abgeleitet von der Cocosfettsäure.

Die WO 94/21226 offenbart Detergensgemische enthaltend Polyhydroxyfettsäureamide, monomere kationische Tenside, Fettalkohole und gegebenenfalls Ölkörper, Haarbehandlungsmittel, die diese Gemische enthalten sowie die Verwendung der Gemische zur Herstellung von Haarbehandlungsmitteln. Konkret offenbart sind Beispiele mit C12/14 Fettsäure-N-alkylpolyhydroxyalkylamide.

Feinteilige, lagerstabile Emulsionen enthaltend unter anderem Fettsäure-N-alkylpolyhydroxyalkylamide und Fettalkohole sind zum Beispiel aus der WO 97/06870 bekannt. Bevorzugt sind hierbei Fettsäure-N-alkylpolyhydroxyalkylamide basierend auf Laurinsäure oder C12/14 Kokosfettsäure.

In der WO 96/27366 sind kosmetische und pharmazeutische Zusammensetzungen offenbart, die als O/W-Emulgatoren Fettsäure-N-alkylglucamide in Konbination mit Fettalkoholen als Coemulgatoren enthalten. Ein Hinweis auf konditionierende Wirkung in der Haarpflege findet sich nicht.

Obwohl mit den bekannten Systemen bereits gute Ergebnisse erzielt werden, bleibt doch ein breiter Raum für Verbesserungen, insbesondere was den Einsatz in kosmetischen Zusammensetzungen zur Haar- und Hautpflege angeht.

Zielsetzung war es somit, Tensidsysteme zu entwickeln, die gut formulierbar sind, höhere Stabilitäten aufweisen, verbesserte konditionierende Effekte zeigen, beispielsweise eine bessere Kämmbarkeit von nassem und trockenem Haar bewirken, statische Aufladung reduzieren, den Griff verbessern sowie Glanz und Farbschutz der Haare bewirken. Insbesondere besteht ein Bedarf an Wirkstoffen, die trotz guter biologischer Abbaubarkeit hervorragende konditionierende Eigenschaften und zudem eine gute Hautverträglichkeit aufweisen. Die Entwicklung von Wirkstoffen sollte auch die Möglichkeit der Bereitstellung der Wirkstoffe in Form von Zusammensetzungen umfassen, wobei diese dann bereits formuliert sein können.

Es wurde gefunden, dass diese Zielsetzung durch Zusammensetzungen gelöst wird, die bestimmte N-Acyl-N-methylglucamine, Fettalkohole und kationische Tenside enthalten.

Gegenstand der Erfindung ist daher eine Zusammensetzung, enthaltend
a) ein oder mehrere N-Methyl-N-acylglucamine, wobei mindestens 50 Gew.-% der N-Methyl-N-acylglucamine eine gesättigte oder ungesättigte C₁₆-, C₁₇- und/oder C₁₈-Acylgruppe aufweisen,
b) einen oder mehrere Fettalkohole,
c) ein oder mehrere monomere kationische Tenside,
d) gegebenenfalls weitere Zusatzstoffe und
e) Wasser.

Erfindungsgemäße Zusammensetzungen weisen eine verbesserte Wärmestabilität, verbesserte Viskositätswerte und im Falle von Haarpflegemitteln ein gutes Haargefühl auf. Die Kombination der Tenside a) bis c) eignet sich insbesondere zur Verdickung von kationischen Emulsionen.

Vorzugsweise enthalten die erfindungsgemäßen Zusammensetzungen
(a) 0,1 bis 5 Gew.-% der Komponente (a),
(b) 0,1 bis 10 Gew.-% der Komponente (b),
(c) 0,1 bis 5 Gew.-% der Komponente (c),
(d) 0 bis 30 Gew.-% der Komponente (d) und
(e) 99,8 bis 50 Gew.-% der Komponente (e),
wobei die Summe der Komponenten (a), (b), (c), (d) und (e) 100 Gew.-% ergibt.

Die erfindungsgemäß verwendeten N-Methyl-N-acylglucamine, bei denen Glucamin eine N-1-Desoxysorbitylgruppe bedeutet, enthalten mindestens 50 Gew.-% N-Methyl-N-acylglucamine, die eine gesättigte oder ungesättigte C₁₆-C₁₈-Acylgruppe enthalten. Bevorzugt liegt der Anteil an N-Methyl-N-acylglucaminen, die eine gesättigte oder ungesättigte C₁₆-C₁₈-Acylgruppe enthalten, bei mindestens 80 Gew.-%, besonders bevorzugt mindestens 90 Gew.-%. Daneben enthalten die erfindungsgemäß verwendeten N-Methyl-N-acylglucamine geringe Anteile an von kurzkettigen und/oder langkettigen Fettsäuren abgeleiteten N-Methyl-N-acylglucaminen, insbesondere solche, welche C₁-C₄-Acyl, C₆-, C₈-, C₁₀- C₁₂-, C₁₄- und/oder C₂₀-Acyl enthalten.

Insbesondere bevorzugt als Komponente a) sind gesättigte N-Methyl-N-acylglucamine der Formel (I), wobei der Acylrest R^{a}CO abgeleitet ist von Palmitinsäure, Stearinsäure, Ölsäure oder Linolsäure:

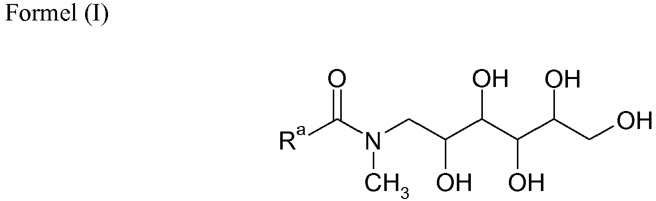

Besonders bevorzugt beträgt der Anteil der Komponente a) in der erfindungsgemäß Zusammensetzung 0,2 Gew.-% bis 3,0 %, ganz besonders bevorzugt 0,5 Gew.-% bis 2 %.

Die N-Methyl-N-acylglucamine können, wie in EP 0 550 637 A1 beschrieben, durch Umsetzung der entsprechenden Fettsäureester oder Fettsäureestergemische mit N-Methylglucamin in Gegenwart eines Hydroxylgruppen oder Alkoxylgruppen aufweisenden Lösungsmittels hergestellt werden. Geeignete Lösungsmittel sind beispielsweise C₁-C₄-Monoalkohole, Ethylenglykol, Propylenglykol, Glycerin sowie alkoxylierte Alkohole. Bevorzugt ist 1,2-Propylenglykol. N-Methylglucamin kann, wie ebenfalls in EP 0 550 637 A1 beschrieben, durch reduktive Aminierung von Glukose mit Methylamin erhalten werden.

Geeignete Fettsäureester, die mit den N-Methylglucaminen zu N-Methyl-N-acylglucaminen umgesetzt werden, sind im Allgemeinen die Methylester, die durch Umesterung aus natürlichen Fetten und Ölen, beispielsweise den Triglyceriden, gewonnen werden.

Geeignete Rohstoffe für die Herstellung der Fettsäuremethylester sind beispielsweise Kokosöl oder Palmöl.

Gegenstand der Erfindung ist auch die Verwendung eines oder mehrerer der erfindungsgemäß eingesetzten N-Methyl-N-acylglucamine zum Verdicken von Emulsionen.

Die erfindungsgemäßen Zusammensetzungen enthalten einen oder mehrere Fettalkohole b), d.h. unverzweigte oder verzweigte Monoalkohole mit einer Alkylgruppe mit 8 bis 22 C-Atomen, und vorzugsweise, bezogen auf die gesamte Zusammensetzung, von 0,1 bis 10,0 Gew.-% an einer oder mehreren dieser Substanzen. Weiterhin bevorzugt sind daher erfindungsgemäße Zusammensetzungen enthaltend einen oder mehrere unverzweigte oder verzweigte Monoalkohole mit einer Alkylgruppe mit 8 bis 22 C-Atomen, und vorzugsweise, bezogen auf die gesamte Zusammensetzung, von 0,1 bis 10,0 Gew.-% an einer oder mehreren dieser Substanzen.

An derartigen Monoalkoholen in Betracht kommen vorzugsweise Laurylalkohol, Stearylalkohol, Cetylalkohol, Guerbetalkohol, Behenylalkohol und deren Mischungen.

Gegenstand der Erfindung ist auch die Verwendung eines oder mehrerer erfindungsgemäß eingesetzter N-Methyl-N-acylglucamine in Kombination mit mindestens einem Fettalkohol (b), wobei das Verhältnis der Komponenten a : b 1 : 9 bis 5 : 5 beträgt.

Die erfindungsgemäße Zusammensetzung enthält weiterhin mindestens ein monomeres kationisches Tensid als Komponente c).

Bevorzugt beträgt der Anteil der Komponente c) in der erfindungsgemäßen Zusammensetzung 0,1 Gew.-% bis 3,0 %, besonders bevorzugt 0,5 Gew.-% bis 2 %.

Geeignete kationische Tenside sind beispielsweise substituierte oder unsubstituierte geradkettige oder verzweigte quartäre Ammoniumsalze vom Typ R¹N(CH₃)₃X, R¹R²N(CH₃)₂X, R¹R²R³N(CH₃)X oder R¹R²R³R⁴NX. Die Reste R¹, R², R³ und R⁴ können vorzugsweise unabhängig voneinander unsubstituiertes Alkyl mit einer Kettenlänge zwischen 8 und 24 C-Atomen, insbesondere zwischen 10 und 18 C-Atomen, Hydroxyalkyl mit 1 bis 4 C-Atomen, Phenyl, C₂- bis C₁₈-Alkenyl, C₇- bis C₂₄-Aralkyl, (C₂H₄O)ₓH, wobei x von 1 bis 3 bedeutet, ein oder mehrere Estergruppen enthaltende Alkylreste oder cyclische quartäre Ammoniumsalze sein. X ist ein geeignetes Anion. Bevorzugt sind (C₈-C₂₂)-Alkyltrimethylammoniumchlorid oder -bromid, besonders bevorzugt Cetyltrimethylammoniumchlorid oder -bromid, Di-(C₈-C₂₂)-Alkyldimethylammoniumchlorid oder -bromid, (C₈-C₂₂)-Alkyldimethylbenzylammoniumchlorid oder -bromid, (C₈-C₂₂)-Alkyl-dimethylhydroxyethylammoniumchlorid, -phosphat, -sulfat, -lactat, besonders bevorzugt Distearyldimethylammoniumchlorid, Di(C₈-C₂₂)-Alkylamidopropyltrimethylammonium-chlorid und -methosulfat.

Bevorzugt als kationische Tenside sind Verbindungen der Formel (II), 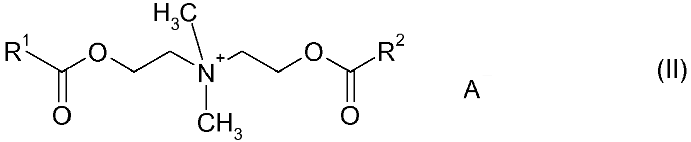 worin
R¹CO und R²CO unabhängig voneinander lineare oder verzweigte gesättigte Acylgruppen mit 18 bis 24 C-Atomen sind,
A⁻ ein Gegenion ist
und die Gesamtmenge an C₁₈₋₂₃-Alkyl-COO-Gruppen, vorzugsweise die Gesamtmenge an C₁₉₋₂₃-Alkyl-COO-Gruppen, bezogen auf alle Gruppen R¹COO- und R²COO-, 40,0 Gew.-% oder mehr, vorzugsweise 42,0 Gew.-% oder mehr, beträgt.

Die Verbindungen der Formel (II), worin R¹CO und R²CO unabhängig voneinander lineare oder verzweigte gesättigte Acylgruppen mit 18 bis 24 C-Atomen sind, können auch derart beschrieben werden, dass R¹ und R² in Formel (II) unabhängig voneinander lineare oder verzweigte Alkylgruppen mit 17 bis 23 C-Atomen sind.

Vorzugsweise sind die Reste R¹ und R² der Verbindungen der Formel (II) unabhängig voneinander lineare Alkylgruppen mit 17 bis 23 C-Atomen.

In einer bevorzugten Ausführungsform der Erfindung ist die Gesamtmenge an C₁₉-Alkyl-COO-, C₂₁-Alkyl-COO- und C₂₃-Alkyl-COO-Gruppen, bezogen auf alle Gruppen R¹COO-und R²COO-, 40,0 Gew.-% oder mehr und vorzugsweise 42,0 Gew.-% oder mehr.

In einer besonders bevorzugten Ausführungsform der Erfindung ist die Gesamtmenge an C₁₉-Alkyl-COO- und C₂₁-Alkyl-COO-Gruppen, bezogen auf alle Gruppen R¹COO- und R²COO-, 40,0 Gew.-% oder mehr und vorzugsweise 42,0 Gew.-% oder mehr.

In einer insbesondere bevorzugten Ausführungsform der Erfindung ist die Gesamtmenge an C₂₁-Alkyl-COO-Gruppen, bezogen auf alle Gruppen R¹COO- und R²COO-, 40,0 Gew.-% oder mehr und vorzugsweise 42,0 Gew.-% oder mehr.

In einer außerordentlich bevorzugten Ausführungsform der Erfindung zeichnen sich die erfindungsgemäßen Zusammensetzungen dadurch aus, dass mehrere Verbindungen der Formel (II) darin enthalten sind und die Menge an C₁₇-Alkyl-COO-Gruppen der Verbindungen der Formel (II), bezogen auf alle Gruppen R¹COO- und R²COO-, von 1,0 bis 45,0 Gew.-% beträgt, die Menge an C₁₉-Alkyl-COO-Gruppen der Verbindungen der Formel (II), bezogen auf alle Gruppen R¹COO- und R²COO-, von 1,0 bis 15,0 Gew.-% beträgt, und die Menge an C₂₁-Alkyl-COO-Gruppen der Verbindungen der Formel (II), bezogen auf alle Gruppen R¹COO- und R²COO-, von 42,0 bis 94,0 Gew.-% beträgt.

In einer überaus bevorzugten Ausführungsform der Erfindung zeichnen sich die erfindungsgemäßen Zusammensetzungen dadurch aus, dass mehrere Verbindungen der Formel (II) darin enthalten sind und die Menge an C₁₇-Alkyl-COO-Gruppen der Verbindungen der Formel (II), bezogen auf alle Gruppen R¹COO- und R²COO-, von 41,0 bis 45,0 Gew.-% beträgt, die Menge an C₁₉-Alkyl-COO-Gruppen der Verbindungen der Formel (II), bezogen auf alle Gruppen R¹COO- und R²COO-, von 9,0 bis 13,0 Gew.-% beträgt, und die Menge an C₂₁-Alkyl-COO-Gruppen der Verbindungen der Formel (II), bezogen auf alle Gruppen R¹COO- und R²COO-, von 42,0 bis 46,0 Gew.-% beträgt.

In einer weiteren überaus bevorzugten Ausführungsform der Erfindung zeichnen sich die erfindungsgemäßen Zusammensetzungen dadurch aus, dass mehrere Verbindungen der Formel (II) darin enthalten sind und die Menge an C₁₇-Alkyl-COO-Gruppen der Verbindungen der Formel (II), bezogen auf alle Gruppen R¹COO- und R²COO-, von 1,0 bis 6,0 Gew.-% beträgt, die Menge an C₁₉-Alkyl-COO-Gruppen der Verbindungen der Formel (II), bezogen auf alle Gruppen R¹COO- und R²COO-, von 12,0 bis 15,0 Gew.-% beträgt, und die Menge an C₂₁-Alkyl-COO-Gruppen der Verbindungen der Formel (II), bezogen auf alle Gruppen R¹COO- und R²COO-, von 79,0 bis 84,0 Gew.-% beträgt.

In einer weiteren überaus bevorzugten Ausführungsform der Erfindung zeichnen sich die erfindungsgemäßen Zusammensetzungen dadurch aus, dass mehrere Verbindungen der Formel (II) darin enthalten sind und die Menge an C₁₇-Alkyl-COO-Gruppen der Verbindungen der Formel (II), bezogen auf alle Gruppen R¹COO- und R²COO-, von 1,0 bis 6,0 Gew.-% beträgt, die Menge an C₁₉-Alkyl-COO-Gruppen der Verbindungen der Formel (II), bezogen auf alle Gruppen R¹COO- und R²COO-, von 1,0 bis 6,0 Gew.-% beträgt, und die Menge an C₂₁-Alkyl-COO-Gruppen der Verbindungen der Formel (II), bezogen auf alle Gruppen R¹COO- und R²COO-, von 90,0 bis 94,0 Gew.-% beträgt.

In einer weiteren bevorzugten Ausführungsform der Erfindung ist die Menge an C₂₃-Alkyl-COO-Gruppen der Verbindungen der Formel (II), bezogen auf alle Gruppen R¹COO- und R²COO-, in den erfindungsgemäßen Zusammensetzungen bis zu 3,0 Gew.-%, vorzugsweise von 0,5 bis 2,5 Gew.-% und besonders bevorzugt von 1,0 bis 2,0 Gew.-%. In einer weiteren überaus bevorzugten Ausführungsform der Erfindung zeichnen sich die erfindungsgemäßen Zusammensetzungen dadurch aus, dass mehrere Verbindungen der Formel (II) darin enthalten sind und die Menge an C₁₇-Alkyl-COO-Gruppen der Verbindungen der Formel (II), bezogen auf alle Gruppen R¹COO- und R²COO-, von 1,0 bis 5,0 Gew.-% beträgt, die Menge an C₁₉-Alkyl-COO-Gruppen der Verbindungen der Formel (1), bezogen auf alle Gruppen R¹COO- und R²COO-, von 6,0 bis 10,0 Gew.-% beträgt, und die Menge an C₂₁-Alkyl-COO-Gruppen der Verbindungen der Formel (II), bezogen auf alle Gruppen R¹COO- und R²COO-, von 86,0 bis 90,0 Gew.-% beträgt. Unter diesen erfindungsgemäßen Zusammensetzungen sind wiederum diejenigen bevorzugt, worin die Menge an C₂₃-Alkyl-COO-Gruppen der Verbindungen der Formel (II), bezogen auf alle Gruppen R¹COO- und R²COO-, von 1,0 bis 5,0 Gew.-% beträgt.

In einer weiteren bevorzugten Ausführungsform der Erfindung enthalten die erfindungsgemäßen Zusammensetzungen eine oder mehrere Verbindungen der Formel (II), worin R¹ und R² lineare oder verzweigte, vorzugsweise lineare, Alkylgruppen mit 21 C-Atomen sind.

Ebenso bevorzugt als kationische Tenside sind Alkyltrimethylammoniumchloride oder Methosulfate der Formel (III),

CₙH₂ₙN⁺(CH₃)₃X⁻ (III)

wobei n bevorzugt 16, 18, 20 oder 22 ist und X⁻ einem Chloridion oder Methosulfation entspricht.

Weiterhin bevorzugt als kationische Tenside sind Tenside der Formel (IV),

[R¹CO-NH-CH₂-CH₂-CH₂-NR²R³R⁴]⁺ X⁻ (IV)

wobei R¹CO ein linearer oder verzweigter , gesättigter oder ungesättigter Acylrest mit 16 bis 32 Kohlenstoffatomen, bevorzugt ein gesättigter, unverzweigter Acylrest mit 18-22 Kohlenstoffatomen ist, R² und R³ Methyl sind, R⁴ H oder Methyl ist und X⁻ ein Anion, vorzugsweise ausgewählt aus Chlorid, Methosulfat, Lactat, Citrat, Glutamat und Glykolat, ist.

Weiterhin Gegenstand der Erfindung ist die Verwendung der Komponenten a) als Verdicker für Emulsionen, bevorzugt zur Verdickung kationischer Emulsionen.

Bei dieser Verwendung werden die Komponenten a) und b) bevorzugt im Gewichtsverhältnis 1 : 9 bis 5 : 5 eingesetzt.

Neben den Komponenten a) b), c) und Wasser e) enthalten die erfindungsgemäßen Zusammensetzungen gegebenenfalls weitere Zusatzstoffe (d).

Zur Verbesserung der Konsistenz der Zusammensetzung kann es vorteilhaft sein, dieser einen oder mehrere kurzkettige Monoalkohole zuzufügen.

In einer weiteren bevorzugten Ausführungsform der Erfindung enthält die Zusammensetzung daher einen oder mehrere Monoalkohole mit einer Alkylgruppe mit 1 bis 7 C-Atomen, und vorzugsweise, bezogen auf die gesamte Zusammensetzung, von 0 bis 10,0 Gew.-% an einer oder mehreren dieser Substanzen.

Bevorzugt als Monoalkohole eingesetzt werden Ethanol, Propanol, Isopropanol, Butanol, Isobutanol und t-Butanol, besonders bevorzugt Isopropanol.

In einer weiteren bevorzugten Ausführungsform der Erfindung enthält die Zusammensetzung ein oder mehrere Polyole mit 3 bis 12 C-Atomen, und vorzugsweise, bezogen auf die gesamte Zusammensetzung, von 0 bis 10,0 Gew.-% an einer oder mehreren dieser Substanzen.

Als mehrwertige Alkohole, d.h. Polyole, bevorzugt sind 1,2-Propylenglykol, 1,3-Propylenglykol, Pentandiol, Hexandiol, Heptandiol, Octandiol, Nonandiol, Decandiol, Undecandiol, Dodecandiol, Diglycerin, Triglycerin, Dipropylenglykol, Tripropylenglykol, Sorbitol, Xylitol, Mannitol und/oder Mischungen derselben. Besonders bevorzugt als mehrwertige Alkohole, d. h. Polyole, sind 1,2-Propylenglykol, 1,3-Propylenglykol, 1,5-Pentandiol, 1,2-Pentandiol, 1,6-Hexandiol, 1,2-Hexandiol, 1,7-Heptandiol, 1,2-Heptandiol, 1,8-Octandiol, 1,2-Octandiol, 1,9-Nonandiol, 1,2-Nonandiol, 1,10-Decandiol, 1,2-Decandiol, 1,11-Undecandiol, 1,2-Undecandiol, 1,12-Dodecandiol, 1,2-Dodecandiol, Diglycerin, Triglycerin, Dipropylenglykol, Tripropylenglykol, Sorbitol, Xylitol, Mannitol und/oder Mischungen derselben.

In einer weiteren bevorzugten Ausführungsform der Erfindung enthalten die erfindungsgemäßen Zusammensetzungen eine oder mehrere Fettsäuren der Formel RCOOH, worin RCO lineare oder verzweigte Acylgruppen mit 8 bis 24 C-Atomen sind, und vorzugsweise, bezogen auf die gesamte Zusammensetzung, von 0,1 bis 5,0 Gew.-% an einer oder mehreren dieser Substanzen.

Die konditionierende Wirkung der erfindungsgemäßen Zusammensetzungen kann durch Zugabe von N-modifizierten Silikonen gesteigert werden.

In einer weiteren bevorzugten Ausführungsform der Erfindung enthalten die erfindungsgemäßen Zusammensetzungen Alkylmethicone, Alkyldimethicone oder eine oder mehrere Amodimethicone. Amodimethicone sind Siloxane Polymere, die mit aminofunktionellen Gruppen gepfropft sind. Amodimethicone sind z.B. unter dem Handelsnamen Dow Corning 2-8566 Amino Fluid (Dow Corning Corporation), Mirasil ADME (Rhodia), SilCare^{®} Silicone SEA (Clariant) oder Wacker-Belsil ADM 1100 (Wacker Chemie AG), haben ein Molekulargewicht zwischen 800 und 260000 g/mol und entsprechen allgemein der Formel (V), 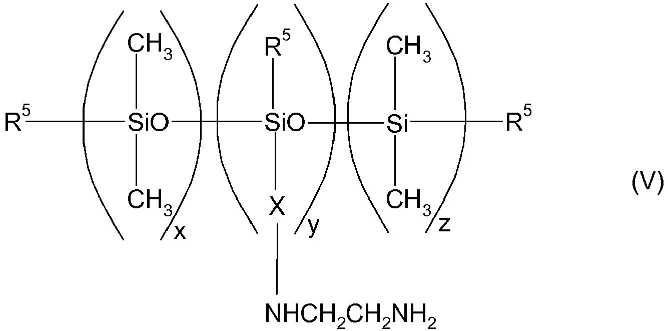 worin
- R⁵: -OH oder -CH₃,
- X: eine lineare oder verzweigte C₁-C₆-Alkylengruppe und
- x, y und z: jeweils unabhängig voneinander eine Zahl von 1 bis 5500, bevorzugt von 50 bis 500, sind.

In einer besonders bevorzugten Ausführungsform der Erfindung enthalten die erfindungsgemäßen Zusammensetzungen eine oder mehrere der oben genannten Verbindungen der Formel (V) und vorzugsweise, bezogen auf die gesamte Zusammensetzung, 0,1 bis 5,0 Gew.-% an einer oder mehreren Verbindungen der Formel (V).

Die Kompatibilität mit weiteren Inhaltsstoffen, sowie das Hautgefühl und der Antistatik-Effekt der Zusammensetzung kann durch Zugabe von einer oder mehreren Verbindungen der Formel (VI) 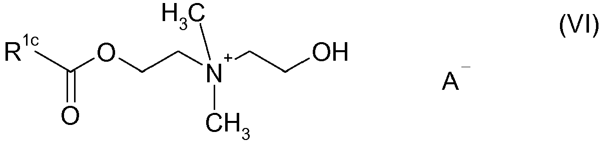 verbessert werden, worin
R^{1c}CO eine lineare oder verzweigte, vorzugsweise eine lineare, des Weiteren bevorzugt eine gesättigte, Acylgruppe mit 18 bis 24 C-Atomen, vorzugsweise mit 18 bis 22 C-Atomen, bedeutet und
A⁻ ein Gegenion ist.

In einer weiteren bevorzugten Ausführungsform der Erfindung enthalten die erfindungsgemäßen Zusammensetzungen eine oder mehrere Verbindungen der Formel (VI), vorzugsweise, bezogen auf die gesamte Zusammensetzung, in einem Anteil von 0,1 bis 5,0 Gew.-%.

Das Gegenion A⁻ der Formel (VI) ist vorzugsweise ausgewählt aus Chlorid, Bromid, Methosulfat (MeSO₄⁻), Tosylat, Phosphat, Sulfat, Hydrogensulfat, Lactat und Citrat und besonders bevorzugt ausgewählt aus Chlorid und Methosulfat.

In einer weiteren bevorzugten Ausführungsform der Erfindung handelt es sich bei den erfindungsgemäßen Zusammensetzungen um Emulsionen.

Die kosmetischen, dermatologischen und pharmazeutischen Zusammensetzungen können als weitere Hilfs- und Zusatzstoffe der Komponente (d) alle üblichen Tenside, Ölkörper, kationischen Polymere, Filmbildner, Verdickungs- und Gelierungsmittel, Überfettungsmittel, antimikrobielle und biogene Wirkstoffe, feuchtigkeitsspendende Mittel, Stabilisatoren, Konservierungsmittel, Perlglanzmittel, Farb- und Duftstoffe, enthalten.

Als weitere Tenside können nichtionische, amphotere und/oder zwitterionische Tenside verwendet werden.

Als nichtionische Tenside bevorzugt sind Fettalkoholethoxylate (Alkylpolyethylenglykole); Alkylphenolpolyethylenglykole; Fettaminethoxylate (Alkylaminopolyethylenglykole); Fettsäureethoxylate (Acylpolyethylenglykole); Polypropylenglykolethoxylate (Pluronics®); Fettsäurealkanolamide, (Fettsäureamidpolyethylenglykole); Saccharoseester; Sorbitester und Sorbitanester und deren Polyglykolether, sowie C₈-C₂₂-Alkylpolyglucoside.

Die Menge der nichtionischen Tenside in den erfindungsgemäßen Zusammensetzungen (z.B. im Falle von Rinse-off-Produkten) liegt bevorzugt im Bereich von 0,1 bis 10,0 Gew.-%, besonders bevorzugt von 0,5 bis 5,0 Gew.-% und insbesondere bevorzugt von 1,0 bis 3,0 Gew.-%.

Weiterhin können die erfindungsgemäßen Zusammensetzungen amphotere Tenside enthalten. Diese können beschrieben werden als Derivate langkettiger sekundärer oder tertiärer Amine, die über eine Alkylgruppe mit 8 bis 18 C-Atomen verfügen und bei denen eine weitere Gruppe substituiert ist mit einer anionischen Gruppe, die die Wasserlöslichkeit vermittelt, so z.B. mit einer Carboxyl-, Sulfat- oder Sulfonat-Gruppe. Bevorzugte Amphotenside sind N-(C₁₂-C₁₈)-Alkyl-β-aminopropionate und N-(C₁₂-C₁₈)-Alkyl-β-iminodipropionate als Alkali- und Mono-, Di- und Trialkylammonium-Salze. Geeignete weitere Tenside sind auch Aminoxide. Es sind dies Oxide tertiärer Amine mit einer langkettigen Gruppe von 8 bis 18 C-Atomen und zwei meist kurzkettigen Alkylgruppen mit 1 bis 4 C-Atomen. Bevorzugt sind hier beispielsweise die C₁₀- bis C₁₈-Alkyldimethylaminoxide und Fettsäureamidoalkyl-dimethylaminoxide.

Eine weitere bevorzugte Gruppe von Tensiden sind Betaintenside, auch zwitterionische Tenside genannt. Diese enthalten im selben Molekül eine kationische Gruppe, insbesondere eine Ammonium-Gruppe und eine anionische Gruppe, die eine CarboxylatGruppe, Sulfat-Gruppe oder Sulfonat-Gruppe sein kann. Geeignete Betaine sind vorzugsweise Alkylbetaine wie Coco-Betain oder Fettsäurealkylamidopropylbetaine, beispielsweise Cocosacylamidopropyldimethylbetain oder die C₁₂-C₁₈-Dimethylaminohexanoate bzw. die C₁₀- bis C₁₈-Acylamidopropandimetylbetaine.

Die Menge der amphoteren Tenside und/oder Betaintenside beträgt bevorzugt von 0,5 bis 20,0 Gew.-% und besonders bevorzugt von 1,0 bis 10,0 Gew.-%.

Bevorzugte Tenside sind, Cocoamidopropylbetain, Alkylbetaine wie Coco-Betain, Natriumcocoylglutamat und Lauroamphoacetat.

In einer bevorzugten Ausführungsform enthalten die erfindungsgemäßen Zusammensetzungen zusätzlich noch als schaumverstärkende Mittel Cotenside aus der Gruppe der Alkylbetaine, Alkylamidobetaine, Aminopropionate, Aminoglycinate, Imidazoliniumbetaine und Sulfobetaine, Aminoxide, Fettsäurealkanolamide und Polyhydroxyamide.

Die Ölkörper können vorteilhafterweise ausgewählt werden aus den Gruppen der Triglyceride, natürliche und synthetische Fettkörper, vorzugsweise Ester von Fettsäuren mit Alkoholen niedriger C-Zahl, z.B. mit Isopropanol, Propylenglycol oder Glycerin, oder Ester von Fettalkoholen mit Alkansäuren niedriger C-Zahl oder mit Fettsäuren oder aus der Gruppe der Alkylbenzoate, sowie natürliche oder synthetische Kohlenwasserstofföle.

In Betracht kommen Triglyceride von linearen oder verzweigten, gesättigten oder ungesättigten, gegebenenfalls hydroxylierten, C₈-C₃₀-Fettsäuren, insbesondere pflanzliche Öle, wie Sonnenblumen-, Mais-, Soja-, Reis-, Jojoba-, Babusscu-, Kürbis-, Traubenkern-, Sesam-, Walnuss-, Aprikosen-, Orangen-, Weizenkeim-, Pfirsichkern-, Makadamia-, Avocado-, Süßmandel-, Wiesenschaumkraut-, Ricinusöl, Olivenöl, Erdnussöl, Rapsöl und Kokosnussöl, sowie synthetische Triglyceridöle, z.B. das Handelsprodukt Myritol^{®} 318. Auch gehärtete Triglyceride sind erfindungsgemäß bevorzugt. Auch Öle tierischen Ursprungs, beispielweise Rindertalg, Perhydrosqualen, Lanolin können eingesetzt werden.

Eine weitere Klasse von erfindungsgemäß bevorzugten Ölkörpern sind die Benzoesäureester von linearen oder verzweigten C₈₋₂₂-Alkanolen, z.B. die Handelsprodukte Finsolv^{®} SB (Isostearylbenzoat), Finsolv^{®} TN (C₁₂-C₁₅-Alkylbenzoat) und Finsolv^{®} EB (Ethylhexylbenzoat).

Eine weitere Klasse von erfindungsgemäß bevorzugten Ölkörpern sind die Dialkylether mit insgesamt 12 bis 36 Kohlenstoffatomen, insbesondere mit 12 bis 24 Kohlenstoffatomen, wie z.B. Di-n-octylether (Cetiol^{®} OE), Di-n-nonylether, Di-n-decylether, Din-undecylether, Di-n-dodecylether, n-Hexyl-n-octylether, n-Octyl-n-decylether, n-Decyl-n-undecylether, n-Undecyl-n-dodecylether und n-Hexyl-n-Undecylether, Di-3-ethyl-decylether, tert.-Butyl-n-octylether, iso-Pentyl-n-octylether und 2-Methyl-pentyl-n-octylether sowie Di-tert.-butylether und Di-iso-pentylether.

Ebenso in Betracht kommen verzweigte gesättigte oder ungesättigte Fettalkohole mit 6 - 30 Kohlenstoffatomen, z.B. Isostearylalkohol, sowie Guerbetalkohole.

Eine weitere Klasse von erfindungsgemäß bevorzugten Ölkörpern sind Hydroxycarbonsäurealkylester. Bevorzugte Hydroxycarbonsäurealkylester sind Vollester der Glykolsäure, Milchsäure, Apfelsäure, Weinsäure oder Zitronensäure. Weitere grundsätzlich geeignete Ester der Hydroxycarbonsäuren sind Ester der β-Hydroxy-propionsäure, der Tartronsäure, der D-Gluconsäure, Zuckersäure, Schleimsäure oder Glucuronsäure. Als Alkoholkomponente dieser Ester eignen sich primäre, lineare oder verzweigte aliphatische Alkohole mit 8 bis 22 C-Atomen. Dabei sind die Ester von C₁₂-C₁₅-Fettalkoholen besonders bevorzugt. Ester dieses Typs sind im Handel erhältlich, z.B. unter dem Handelsnamen Cosmacol^{®} der EniChem, Augusta Industriale.

Eine weitere Klasse von erfindungsgemäß bevorzugten Ölkörpern sind Dicarbonsäureester von linearen oder verzweigten C₂-C₁₀-Alkanolen, wie Di-n-butyladipat (Cetiol^{®} B), Di-(2-ethylhexyl)-adipat und Di-(2-ethylhexyl)-succinat sowie Diolester wie Ethylenglykoldioleat, Ethylenglykoldiisotridecanoat, Propylenglycol-di-(2-ethylhexanoat), Propylenglycoldiisostearat, Propylenglycol-di-pelargonat, Butandiol-di-isostearat und Neopentylglycoldicaprylat sowie Diisotridecylacelaat.

Ebenso bevorzugte Ölkörper sind symmetrische, unsymmetrische oder cyclische Ester der Kohlensäure mit Fettalkoholen, Glycerincarbonat oder Dicaprylylcarbonat (Cetiol^{®} CC).

Eine weitere Klasse von erfindungsgemäß bevorzugten Ölkörpern sind die Ester von Dimeren ungesättigter C₁₂-C₂₂-Fettsäuren (Dimerfettsäuren) mit einwertigen linearen, verzweigten oder cyclischen C₂-C₁₈-Alkanolen oder mit mehrwertig linearen oder verzweigten C₂-C₆-Alkanolen.

Eine weitere Klasse von erfindungsgemäß bevorzugten Ölkörpern sind Kohlenwasserstofföle, zum Beispiel solche mit linearen oder verzweigten, gesättigten oder ungesättigten C₇-C₄₀-Kohlenstoffketten, beispielsweise Vaseline, Dodecan, Isododecan, Cholesterol, Lanolin, synthetische Kohlenwasserstoffe wie Polyolefine, insbesondere Polyisobuten, hydriertes Polyisobuten, Polydecan, sowie Hexadecan, Isohexadecan, Paraffinöle, Isoparaffinöle, z.B. die Handelsprodukte der Permethyl^{®}-Serie, Squalan, Squalen, und alicyclische Kohlenwasserstoffe, z.B. das Handelsprodukt 1,3-Di-(2-ethylhexyl)-cyclohexan (Cetiol^{®} S), Ozokerit, und Ceresin.

An Silikonölen bzw. -wachsen stehen vorzugsweise zur Verfügung Dimethylpolysiloxane und Cyclomethicone, Polydialkylsiloxane R₃SiO(R₂SiO)ₓSiR₃, wobei R für Methyl oder Ethyl, besonders bevorzugt für Methyl, steht und x für eine Zahl von 2 bis 500 steht, beispielsweise die unter den Handelsnamen VICASIL (General Electric Company), DOW CORNING 200, DOW CORNING 225, DOW CORNING 200 (Dow Corning Corporation), erhältlichen Dimethicone, sowie die unter SilCare^{®} Silicone 41M65, SilCare^{®} Silicone 41M70, SilCare^{®} Silicone 41M80 (Clariant) erhältlichen Dimethicone, Stearyldimethylpolysiloxan, C₂₀-C₂₄-Alkyl-dimethylpolysiloxan, C₂₄-C₂₈-lkyl-dimethyl-polysiloxan, aber auch die unter SilCare^{®} Silicone 41M40, SilCare^{®} Silicone 41M50 (Clariant) erhältlichen Methicone, weiterhin Trimethylsiloxysilicate [(CH₂)₃SiO)_{1/2}]ₓ[SiO₂]_{y}, wobei x für eine Zahl von 1 bis 500 und y für eine Zahl von 1 bis 500 steht, Dimethiconole R₃SiO[R₂SiO]ₓSiR₂OH und HOR₂SiO[R₂SiO]ₓSiR₂OH, wobei R für Methyl oder Ethyl und x für eine Zahl bis zu 500 steht, Polyalkylarylsiloxane, beispielsweise die unter den Handelsbezeichnungen SF 1075 METHYLPHENYL FLUID (General Electric Company) und 556 COSMETIC GRADE PHENYL TRIMETHICONE FLUID (Dow Corning Corporation) erhältlichen Polymethylphenylsiloxane, Polydiarylsiloxane, Silikonharze, cyclische Silikone und amino-, fettsäure-, alkohol-, polyether-, epoxy-, fluor- und/oder alkylmodifizierte Silikonverbindungen, sowie Polyethersiloxan-Copolymere.

Als kationische Polymere eignen sich die unter der INCI-Bezeichnung "Polyquaternium" bekannten, insbesondere Polyquaternium-31, Polyquaternium-16, Polyquaternium-24, Polyquaternium-7, Polyquaternium-22, Polyquaternium-39, Polyquaternium-28, Polyquaternium-2, Polyquaternium-10, Polyquaternium-11, sowie Polyquaternium 37&mineral oil&PPG trideceth (Salcare SC95), PVP-dimethylaminoethylmethacrylat-Copolymer, Guarhydroxypropyltriammoniumchloride, sowie Calciumalginat und Ammoniumalginat. Des Weiteren können eingesetzt werden kationische Cellulosederivate; kationische Stärke; Copolymere von Diallylammoniumsalzen und Acrylamiden; quaternierte Vinylpyrrolidon/Vinylimidazol-Polymere, Kondensationsprodukte von Polyglykolen und Aminen; quaternierte Kollagenpolypeptide; quaternierte Weizenpolypeptide; Polyethylenimine; kationische Siliconpolymere, wie z.B. Amidomethicone; Copolymere der Adipinsäure und Dimethylaminohydroxypropyldiethylentriamin; Polyaminopolyamid und kationische Chitinderivate, wie beispielsweise Chitosan.

Die erfindungsgemäßen Zusammensetzungen können einen oder mehrere der oben genannten kationischen Polymere in Mengen von 0,1 bis 5,0 Gew.-%, vorzugsweise von 0,2 bis 3,0 Gew.-% und besonders bevorzugt von 0,5 bis 2,0 Gew.-%, bezogen auf die fertigen Zusammensetzungen, enthalten.

Des Weiteren können die erfindungsgemäßen Zusammensetzungen Filmbildner enthalten, die je nach Anwendungszweck ausgewählt sind aus Salzen der Phenylbenzimidazolsulfonsäure, wasserlöslichen Polyurethanen, beispielsweise C₁₀-Polycarbamylpolyglycerylester, Polyvinylalkohol, Polyvinylpyrrolidoncopolymeren wie PVP/Hexandecene oder PVP/Eicosene Copolymer, beispielsweise Vinylpyrrolidon/Vinylacetatcopolymer, wasserlöslichen Acrylsäurepolymeren/Copolymeren bzw. deren Estern oder Salzen, beispielsweise Partialestercopolymere der Acryl/Methacrylsäure und Polyethylenglykolethern von Fettalkoholen, wie Acrylat/Steareth-20-Methacrylat Copolymer, wasserlöslicher Cellulose, beispielsweise Hydroxymethylcellulose, Hydroxyethylcellulose, Hydroxypropylcellulose, wasserlöslichen Quaterniums, Polyquaterniums, Carboxyvinyl-Polymeren, wie Carbomere und deren Salze, Polysacchariden, beispielsweise Polydextrose und Glucan, Vinylacetat/Crotonat, beispielsweise unter dem Handelsnamen Aristoflex^{®} A 60 (Clariant) erhältlich, sowie polymeren Aminoxiden, beispielsweise unter den Handelsnamen Diaformer Z-711, 712, 731, 751 erhältliche Vertreter.

Die erfindungsgemäßen Zusammensetzungen können einen oder mehrere Filmbildner in Mengen von 0,1 bis 10,0 Gew.-%, vorzugsweise von 0,2 bis 5,0 Gew.-% und besonders bevorzugt von 0,5 bis 3,0 Gew.-%, bezogen auf die fertigen Zusammensetzungen, enthalten.

Die gewünschte Viskosität der Zusammensetzungen kann durch Zugabe von Verdickern und Gelierungsmittel eingestellt werden. In Betracht kommen vorzugsweise Celluloseether und andere Cellulosederivate (z.B. Carboxymethylcellulose, Hydroxyethylcellulose), Gelatine, Stärke und Stärkederivate, Natriumalginate, Fettsäurepolyethylenglykolester, Agar-Agar, Traganth oder Dextrinderivate, insbesondere Dextrinester. Desweiteren eignen sich Metallsalze von Fettsäuren, bevorzugt mit 12 bis 22 C-Atomen, beispielsweise Natriumstearat, Natriumpalmitat, Natriumlaurat, Natriumarachidate, Natriumbehenat, Kaliumstearat, Kaliumpalmitat, Natriummyristat, Aluminiummonostearat, Hydroxyfettsäuren, beispielsweise 12-Hydroxystearinsäure, 16-Hydroxyhexadecanoylsäure; Fettsäureamide; Fettsäurealkanolamide; Dibenzalsorbit und alkohollösliche Polyamide und Polyacrylamide oder Mischungen solcher. Weiterhin können vernetzte und unvernetzte Polyacrylate wie Carbomer, Natriumpolyacrylate oder sulfonsäurehaltige Polymere wie Ammoniumacryloyldimethyltaurate/VP-Copolymer Verwendung finden.

Bevorzugt enthalten die erfindungsgemäßen Zusammensetzungen von 0,01 bis 10,0 Gew.-%, besonders bevorzugt von 0,1 bis 5,0 Gew.-%, insbesondere bevorzugt von 0,2 bis 3,0 Gew.-% und ganz besonders bevorzugt von 0,4 bis 2,0 Gew.-% an Verdickern bzw. Geliermitteln.

Als Überfettungsmittel können vorzugsweise Lanolin und Lecithin, nicht ethoxylierte und polyethoxylierte oder acylierte Lanolin- und Lecithinderivate, Polyolfettsäureester, Mono-, Di- und Triglyceride und/oder Fettsäurealkanolamide, wobei die letzteren gleichzeitig als Schaumstabilisatoren dienen, verwendet werden, die bevorzugt in Mengen von 0,01 bis 10,0 Gew.-%, besonders bevorzugt von 0,1 bis 5,0 Gew.-% und insbesondere bevorzugt von 0,5 bis 3,0 Gew.-% eingesetzt werden.

An antimikrobiellen Wirkstoffen kommen Cetyltrimethylammoniumchlorid, Cetylpyridiniumchlorid, Benzethoniumchlorid, Diisobutylethoxyethyldimethylbenzylammoniumchlorid, Natrium-N-Laurylsarcosinat, Natrium-N-Palmethylsarcosinat, Lauroylsarcosin, N-Myristoylglycin, Kalium-N-Laurylsarcosin, Trimethylammoniumchlorid, Natriumaluminiumchlorohydroxylactat, Triethylcitrat, Tricetylmethylammoniumchlorid, 2,4,4'-Trichloro-2'-hydroxydiphenylether (Triclosan), Phenoxyethanol, 1,5-Pentandiol, 1,6-Hexandiol, 3,4,4'-Trichlorocarbanilid (Triclocarban), Diaminoalkylamid, beispielsweise L-Lysinhexadecylamid, Citratschwermetallsalze, Salicylate, Piroctose, insbesondere Zinksalze, Pyrithione und deren Schwermetallsalze, insbesondere Zinkpyrithion, Zinkphenolsulfat, Farnesol, Ketoconazol, Oxiconazol, Bifonazole, Butoconazole, Cloconazole, Clotrimazole, Econazole, Enilconazole, Fenticonazole, Isoconazole, Miconazole, Sulconazole, Tioconazole, Fluconazole, Itraconazole, Terconazole, Naftifine und Terbinafine, Selendisulfid und Octopirox, Iodopropynylbutylcarbamat, Methylchloroisothiazolinon, Methylisothiazolinon, Methyldibromo Glutaronitril, AgCl, Chloroxylenol, Na-Salz von Diethylhexylsulfosuccinat, Natriumbenzoat, sowie Phenoxyethanol, Benzylalkohol, Phenoxyisopropanol, Parabene, bevorzugt Butyl-, Ethyl-, Methyl- und Propylparaben, sowie deren Na-Salze, Pentandiol, 1,2-Octandiol, 2-Bromo-2-Nitropropan-1,3-diol, Ethylhexylglycerin, Benzylalkohol, Sorbinsäure, Benzoesäure, Milchsäure, Imidazolidinylharnstoff, Diazolidinylharnstoff, Dimethyloldimethylhydantoin (DMDMH), Na-Salz von Hydroxymethylglycinat, Hydroxyethylglycin der Sorbinsäure und Kombinationen dieser Wirksubstanzen zum Einsatz.

Die erfindungsgemäßen Zusammensetzungen enthalten die antimikrobiellen Wirkstoffe bevorzugt in Mengen von 0,001 bis 5,0 Gew.-%, besonders bevorzugt von 0,01 bis 3,0 Gew.-% und insbesondere bevorzugt von 0,1 bis 2,0 Gew.-%, bezogen auf die fertigen Zusammensetzungen.

Die erfindungsgemäßen Zusammensetzungen können des Weiteren biogene Wirkstoffe ausgewählt aus Pflanzenextrakten, wie beispielsweise Aloe Vera, sowie Lokalanästhetika, Antibiotika, Antiphlogistika, Antiallergika, Corticosteroide, Sebostatika, Bisabolol^{®}, Allantoin^{®}, Phytantriol^{®}, Proteine, Vitamine ausgewählt aus Niacin, Biotin, Vitamin B2, Vitamin B3, Vitamin B6, Vitamin B3 Derivaten (Salzen, Säuren, Estern, Amiden, Alkoholen), Vitamin C und Vitamin C Derivaten (Salzen, Säuren, Estern, Amiden, Alkoholen), bevorzugt als Natriumsalz des Monophosphorsäureesters der Ascorbinsäure oder als Magnesiumsalz des Phosphorsäureesters der Ascorbinsäure, Tocopherol und Tocopherolacetat, sowie Vitamin E und/oder dessen Derivate enthalten.

Die erfindungsgemäßen Zusammensetzungen können biogene Wirkstoffe bevorzugt in Mengen von 0,001 bis 5,0 Gew.-%, besonders bevorzugt von 0,01 bis 3,0 Gew.-% und insbesondere bevorzugt von 0,1 bis 2,0 Gew.-%, bezogen auf die fertigen Zusammensetzungen, enthalten.

Die erfindungsgemäßen Zusammensetzungen können Adstringentien, bevorzugt Magnesiumoxid, Aluminiumoxid, Titandioxid, Zirkondioxid und Zinkoxid, Oxidhydrate, bevorzugt Aluminiumoxidhydrat (Böhmit) und Hydroxide, bevorzugt von Calcium, Magnesium, Aluminium, Titan, Zirkon oder Zink, sowie Aluminiumchlorohydrate bevorzugt in Mengen von 0 bis 50,0 Gew.-%, besonders bevorzugt in Mengen von 0,01 bis 10,0 Gew.-% und insbesondere bevorzugt in Mengen von 0,1 bis 10,0 Gew.-% enthalten. Als deodorierende Stoffe bevorzugt sind Allantoin und Bisabolol. Diese werden vorzugsweise in Mengen von 0,0001 bis 10,0 Gew.-% eingesetzt.

Als feuchtigkeitsspendende Substanz stehen beispielsweise Isopropylpalmitat, Glycerin und/oder Sorbitol zu Verfügung.

Als Stabilisatoren können Metallsalze von Fettsäuren, wie z.B. Magnesium-, Aluminium- und/oder Zinkstearat eingesetzt werden, bevorzugt in Mengen von 0,1 bis 10,0 Gew.-%, vorzugsweise von 0,5 bis 8,0 Gew.-% und besonders bevorzugt von 1,0 bis 5,0 Gew.-%, bezogen auf die fertigen Zusammensetzungen.

Als Konservierungsmittel eignen sich die im betreffenden Annex der europäischen Kosmetikgesetzgebung aufgeführten Konservierungsmittel, beispielsweise Phenoxyethanol, Benzylalkohol, Parabene, Benzoesäure und Sorbinsäure, besonders gut geeignet ist beispielsweise 1,3-Bis(hydroxymethyl)-5,5-dimethylimidazolidine-2,4-dione (Nipaguard^{®} DMDMH).

Als perlglanzgebende Komponente bevorzugt geeignet sind Fettsäuremonoalkanolamide, Fettsäuredialkanolamide, Monoester oder Diester von Alkylenglykolen, insbesondere Ethylenglykol und/oder Propylenglykol oder dessen Oligomere, mit höheren Fettsäuren, wie z.B. Palmitinsäure, Stearinsäure und Behensäure, Monoester oder Polyester von Glycerin mit Carbonsäuren, Fettsäuren und deren Metallsalze, Ketosulfone oder Gemische der genannten Verbindungen. Besonders bevorzugt sind Ethylenglykoldistearate und/oder Polyethylenglykol-distearate mit durchschnittlich 3 Glykoleinheiten.

Sofern die erfindungsgemäßen Zusammensetzungen perlglanzgebende Verbindungen enthalten, sind diese bevorzugt in einer Menge von 0,1 bis 15,0 Gew.-% und besonders bevorzugt in einer Menge von 1,0 bis 10,0 Gew.-% in den erfindungsgemäßen Zusammensetzungen enthalten.

Als Duft- bzw. Parfümöle können einzelne Riechstoffverbindungen, z.B. die synthetischen Produkte vom Typ der Ester, Ether, Aldehyde, Ketone, Alkohole und Kohlenwasserstoffe verwendet werden. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 C-Atomen, Citral, Citronellal, Citronellyloxyacetaldehyd, Cycllamenaldehyd, Hydroxycitronellal, Lilial und Bourgeonal, zu den Ketonen z.B. die Ionone, alpha-Isomethylionon und Methyl-cedrylketon, zu den Alkoholen Anethol, Citronellol, Eugenol, Geranion, Linalol, Phenylethylalkohol und Terpineol, zu den Kohlenwasserstoffen gehören hauptsächlich die Terpene und Balsame. Bevorzugt werden Mischungen verschiedener Riechstoffe verwendet, die gemeinsam eine ansprechende Duftnote erzeugen.

Parfümöle können auch natürliche Riechstoffgemische enthalten, wie sie aus pflanzlichen oder tierischen Quellen zugänglich sind, z.B. Pinien-, Citrus-, Jasmin-, Lilien-, Rosen-, oder Ylang-Ylang-Öl. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl und Ladanumöl.

Die erfindungsgemäßen Zusammensetzungen sind vorzugsweise auf einen pH Wert im Bereich von 2 bis 12, bevorzugt im Bereich von 3 bis 9, eingestellt.

Als Säuren oder Laugen zur pH-Wert Einstellung werden vorzugsweise Mineralsäuren, insbesondere HCl, anorganische Basen, insbesondere NaOH oder KOH, oder organische Säuren, insbesondere Zitronensäure, verwendet.

Der Gesamtanteil an weiteren Zusatzstoffen der Komponente (d) in den Mitteln bzw. in den erfindungsgemäßen Zusammensetzungen beträgt bevorzugt von 1,0 bis 25,0 Gew.-% und besonders bevorzugt von 2,0 bis 20,0 Gew.-%.

In einer bevorzugten Ausführungsform der Erfindung handelt es sich bei den erfindungsgemäßen Zusammensetzungen um kosmetische, dermatologische oder pharmazeutische Zusammensetzungen.

Vorzugsweise handelt es sich bei den erfindungsgemäßen kosmetischen dermatologischen oder pharmazeutischen Zusammensetzungen um Pflegemittel für die Haut und um Haarbehandlungsmittel.

Beispiele für bevorzugte erfindungsgemäße kosmetische, dermatologische oder pharmazeutische Zusammensetzungen sind Hautpflegemittel wie Cremes, Lotionen, Tagescremes, Nachtcremes, Pflegecremes, Nährcremes, Bodylotions und Salben.

In einer weiteren bevorzugten Ausführungsform der Erfindung handelt es sich bei den erfindungsgemäßen Zusammensetzungen um Öl-in-Wasser Emulsionen, vorzugsweise um Öl-in-Wasser Emulsionen zur Behandlung oder Pflege der Haut.

In einer besonders bevorzugten Ausführungsform der Erfindung handelt es sich bei den erfindungsgemäßen Zusammensetzungen um Zusammensetzungen zur Behandlung oder Pflege von Haaren, insbesondere Haarnachbehandlungsmittel wie rinse-off Haarconditionierer, Cremespülungen, Klarspülungen, Haarkuren, Haarfärbe- und Haartönungsmittel, Dauerwellmittel, Haarconditionierer in Aerosol-, Spray- und Fluidform.

Die erfindungsgemäßen Zusammensetzungen, insbesondere die erfindungsgemäßen kosmetischen, dermatologischen oder pharmazeutischen Zusammensetzungen, sind in vorteilhafter Weise zur Behandlung oder Pflege der Haut geeignet.

Weiterer Gegenstand der vorliegenden Erfindung ist daher die Verwendung der erfindungsgemäßen Zusammensetzungen, insbesondere der erfindungsgemäßen kosmetischen, dermatologischen oder pharmazeutischen Zusammensetzungen, zur Behandlung oder Pflege der Haut.

Die erfindungsgemäßen Zusammensetzungen, insbesondere die erfindungsgemäßen kosmetischen, dermatologischen oder pharmazeutischen Zusammensetzungen, sind des Weiteren in vorteilhafter Weise zur Behandlung oder Pflege von Haaren geeignet.

Weiterer Gegenstand der vorliegenden Erfindung ist daher die Verwendung der erfindungsgemäßen Zusammensetzungen, insbesondere der erfindungsgemäßen kosmetischen, dermatologischen oder pharmazeutischen Zusammensetzungen, zur Behandlung oder Pflege von Haaren.

Bevorzugt ist die Verwendung der erfindungsgemäßen kosmetischen, dermatologischen oder pharmazeutischen Zusammensetzungen als Haarnachbehandlungsmittel, insbesondere zum Konditionieren von Haaren.

Die nachfolgenden Beispiele dienen der Erläuterung der Erfindung, ohne sie jedoch darauf einzuschränken.

### Beispiele

Die in den Beispielen angegebenen %-Angaben sind Gewichtsprozent (Gew.-%), sofern nicht explizit anders angegeben.

Die im folgenden beschriebene N-Acyl-N-Methylglucamine wurde nach EP 0 550 637 aus den korrespondierenden Fettsäuremethylestern und N-Methylglucamid in Gegenwart von 1,2-Propylenglykol als Lösemittel hergestellt und als Feststoff bestehend aus Aktivsubstanz, d.h. N-Methyl-N-acylglucaminen, und 1,2-Propylenglykol erhalten.

| Herstellbeispiel | Methylester | Aktivsubstanz (%) | 1,2-Propylenglykol (%) | Schmelzpunkt |
|---|---|---|---|---|
| 1 | C12/14 | 90 | 10 | 85 |
| 2 | C16/18 | 80 | 20 | 65 |
| 3 | C20/22 | 80 | 20 | 120 |

Die Viskositäten wurden mit einem Brookfield-Viskosimeter Model DV II, den Spindeln aus dem Spindelset RV bei 20 Umdrehungen / Minute und 20 °C gemessen. Es werden die Spindeln 1 bis 7 aus dem Spindelset RV verwendet. Unter diesen Messbedingungen wird Spindel 1 für Viskositäten von maximal 500 mPa·s, Spindel 2 für Viskositäten von maximal 1 000 mPa·s, Spindel 3 für Viskositäten von maximal 5 000 mPa·s, Spindel 4 für Viskositäten von maximal 10 000 mPa·s, Spindel 5 für Viskositäten von maximal 20 000 mPa·s, Spindel 6 für Viskositäten von maximal 50 000 mPa·s und Spindel 7 für Viskositäten von maximal 200 000 mPa·s gewählt.

Die folgenden Cremespülungsprodukte wurden aus Emulgator, Cetearylalkohol und Behentrimoniumchlorid (Genmin^{®} KDMP, Clariant) hergestellt.

### Herstellung:

Emulgator (Einsatz nach Aktivsubstanz, 1,5 %) und Cetearylalkohol (3 %) wurden bei 80 °C aufgeschmolzen, parallel wurde das Behentrimoniumchlorid (1 % Aktivsubstanz) bei 80 °C in Wasser gelöst. Die beiden Phasen wurden unter Rühren vereinigt und die entstehende Cremespülung unter Rühren abgekühlt und der pH-Wert mit Zitronensäure auf pH = 4,0 eingestellt.

| Beispiel | Emulgator | Viskosität bei 20 °C | Aussehen |
|---|---|---|---|
| Vergleichsbeispiel 1 | Ceteareth-3 | 5500 | Weiße Emulsion |
| Vergleichsbeispiel 2 | Herstellbeispiel 1 | 900 | Weiße , niedrigviskose Emulsion |
| Beispiel 1 | Herstellbeispiel 2 | 7400 | Weiße Emulsion |
| Vergleichsbeispiel 3 | Herstellbeispiel 3 | 7100 | Weiße Emulsion mit Inhomogenitäten |

Die Produkte wurden in einem trainierten Sensorikpanel mit 5 Teilnehmern bezüglich der unten genannten Parameter bewertet. ++ = sehr gut, + = gut o = befriedigend, - = ausreichend -- = unbefriedigend bewertet und die Parameter nach Mittelwertbildung bewertet qualitativ gegen den Standard (Vergleichsbeispiel 1) bewertet. Hierbei wurde nach Waschen der Haare in einem Halbseitentest zwei der Testprodukte auf jeweils eine Kopfhälfte aufgetragen, mit Wasser ausgespült und die Haareigenschaften währen des Ausspülens, während der Trocknung und im Trockenen bewertet.

| Beispiel | Haarglätte beim Ausspülen | Haargefühl während des Trocknens | Haargefühl im Trockenen |
|---|---|---|---|
| Vergleichsbeispiel 1 | o (Standard) | o (Standard) | o (Standard) |
| Vergleichsbeispiel 2 | o (keine Verbesserung zum Standard) | + (etwas weicher) | o (keine Verbesserung zum Standard) |
| Beispiel 1 | + (weicher, gutes detangling) | ++ (weicher) | + (besser als Vergleichsbeispiel 1 auf den Haarspitzen) |
| Vergleichsbeispiel 3 | Inhomogene Emulsion | Inhomogene Emulsion | Inhomogene Emulsion |

Die erfindungsgemäßen Zusammensetzungen (Beispiel 1) zeigen gegenüber dem Stand der Technik (Henkel C12/14 Glucamid, Vergleichsbeispiel 2) deutlich verbesserte Viskositäten auf. Außerdem werden die Konditioniereigenschaften bei Verwendung der erfindungsgemäßen C16-18 N-Methyl N-Acylglucamine gegenüber den Vergleichsbeispielen deutlich verbessert, speziell wird die Weichheit des Haares während der Trocknungsphase verbessert.

## Patentansprüche

1. Zusammensetzung, enthaltend
a) ein oder mehrere N-Methyl-N-acylglucamine, wobei mindestens 50 Gew.-% der N-Methyl-N-acylglucamine eine C₁₆-, C₁₇- und/oder C₁₈-Acylgruppe aufweisen,
b) einen oder mehrere Fettalkohole,
c) ein oder mehrere monomere kationische Tenside,
d) gegebenenfalls weitere Zusatzstoffe und
e) Wasser.

2. Zusammensetzung gemäß Anspruch 1, wobei mindestens 50 Ges.-% der N-Methyl-N-acylglucamine Verbindungen der Formal (I) entsprechen, 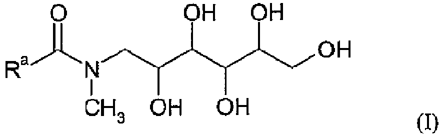 wobei R^{a}-CO von Palmitinsäure, Stearinsäure, Ölsäure oder Linolsäure abgeleitet ist.

3. Zusammensetzung gemäß Anspruch 1 oder 2, enthaltend
(a) 0,1 bis 5 Gew.-% der Komponente (a),
(b) 0,1 bis 10 Gew.% der Komponente (b),
(c) 0,1 bis 5 Gew.-% der Komponente (c),
(d) 0 bis 30 Gew.-% der Komponente (d) und
(e) 99,8 bis 50 Gew.-% der Komponente (e).
wobei die Summe der Komponenten (a), (b), (c), (d) und (e) 100 Gew.-% ergibt

4. Zusammensetzung gemäß einem der Ansprüche 1 bis 3, enthaltend
(a) 0,2 bis 3 Gew.-% der Komponente (a),
(b) 0,1 bis 10 Gew.-% der Komponente (c),
(c) 0,1 bis 3 Gew.-% der Komponente (b),
(d) 0 bis 30 Gew.-% der Komponente (d) und
(e) 99,6 bis 54 Gew.% der Komponente (e).
wobei die Summe der Komponenten (a), (b), (c), (d) und (e) 100 Gew.-% ergibt.

5. Zusammensetzung gemäß einem der Ansprüche 1 bis 4, wobei die Komponente a) mindestens 80 Gew.-% an N-Methyl-N-acylglucaminen enthält, die eine C₁₆-C₁₇-und/oder C₁₈-Acylgruppe aufweisen.

6. Zusammensetzung gemäß einem der Ansprüche 1 bis 5 enthaltend ein oder mehrere kationische Tenside der Formel (II), 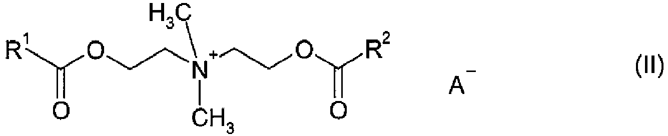 worin
R¹CO und R²CO unabhängig voneinander lineare oder verzweigte gesättigte Acylgruppen mit 18 bis 24 C-Atomen sind und
A- ein Gegenion ist
und die Gesamtmenge an C₁₈₋₂₃-Alkyl-COO-Gruppen, bezogen auf alle Gruppen R¹COO- und R²COO-, 40,0 Gew.-% oder mehr ist.

7. Zusammensetzung gemäß einem der Ansprüche 1 bis 6, enthaltend ein oder mehrere kationische Tenside der Formel (III),
CₙH₂ₙN⁺(CH₃)₃ X⁻ (III)
wobei n 16, 18, 20 oder 22 ist und X⁻ einem Chloridion oder Methosulfation entspricht.

8. Zusammensetzung gemäß einem der Ansprüche 1 bis 7, enthaltend ein oder mehrere kationische Tenside der Formel (IV),
[R¹CO-NH-CH₂-CH₂-CH₂-NR²R³R⁴]⁺ X⁻ (IV)
wobei R¹CO ein linearer oder verzweigter , gesättigter oder ungesättigter Acylrest mit 16 bis 32 Kohlenstoffatomen ist, R² und R³ Methyl sind, R⁴ H oder Methyl ist und X⁻ ein Anion ausgewählt aus Chlorid, Methosulfat, Lactat, Citrat, Glutamat und Glykolat ist.

9. Zusammensetzung gemäß einem der Ansprüche 1 bis 8, wobei die Komponente c) einen oder mehreren Fettalkoholen aus der Gruppe Laurylalkohol, Stearylalkohol, Cetylalkohol, Guerbetalkohol und Behenylalkohol enthält.

10. Kosmetische, dermatologische oder pharmazeutische Zusammensetzung gemäß einem der Ansprüche 1 bis 9.

11. Zusammensetzung gemäß Anspruch 10 in Form einer Emulsion.

12. Kosmetische Zusammensetzung gemäß Anspruch 10 oder 11 in Form eines Haarnachbehandlungsmittels.

13. Zusammensetzung gemäß einem der Ansprüche 1 bis 11 in Form einer Creme oder Lotion.

14. Verwendung eines oder mehrerer N-Methyl-N-acylglucamine gemäß Anspruch 1 oder 2 zum Verdicken von Emulsionen, wobei das oder die N-Methyl-N-acylglucamine a) zusammen mit mindestens einem Fettalkohol b) gemäß Anspruch 1 oder 9 eingesetzt werden und das Verhältnis der Komponenten a : b 1 : 9 bis 5 : 5 beträgt.

## Claims

1. A composition comprising
a) one or more N-methyl-N-acylglucamines, where at least 50% by weight of the N-methyl-N-acylglucamines have a C₁₆-, C₁₇- and/or C₁₈-acyl group,
b) one or more fatty alcohols,
c) one or more monomeric cationic surfactants,
d) optionally further additives and
e) water.

2. The composition as claimed in claim 1, wherein at least 50% by weight of the N-methyl-N-acylglucamines are compounds of the formula (I) 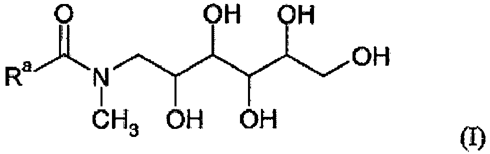 where R^{a}-CO is derived from palmitic acid, stearic acid, oleic acid or linoleic acid.

3. The composition as claimed in claim 1 or 2, comprising
(a) 0.1% to 5% by weight of component (a),
(b) 0.1% to 10% by weight of component (b),
(c) 0.1% to 5% by weight of component (c),
(d) 0% to 30% by weight of component (d) and
(e) 99.8% to 50% by weight of component (e),
where the sum total of components (a), (b), (c), (d) and (e) is 100% by weight.

4. The composition as claimed in any of claims 1 to 3, comprising
(a) 0.2% to 3% by weight of component (a),
(b) 0.1% to 10% by weight of component (c),
(c) 0.1% to 3% by weight of component (b),
(d) 0% to 30% by weight of component (d) and
(e) 99.6% to 54% by weight of component (e),
where the sum total of components (a), (b), (c), (d) and (e) is 100% by weight.

5. The composition as claimed in any of claims 1 to 4, wherein component a) contains at least 80% by weight of N-methyl-N-acylglucamines having a C₁₆-C₁₇-and/or C₁₈-acyl group.

6. The composition as claimed in any of claims 1 to 5, comprising one or more cationic surfactants of the formula (II) 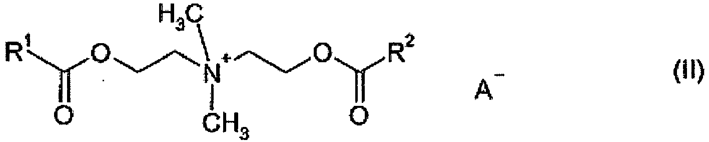 in which
R¹CO and R²CO are each independently linear or branched saturated acyl groups having 18 to 24 carbon atoms,
A⁻ is a counterion
and the total amount of C₁₈₋₂₃-alkyl-COO- groups, based on all the R¹COO- and R²COO- groups, is 40.0% by weight or more.

7. The composition as claimed in any of claims 1 to 6, comprising one or more cationic surfactants of the formula (III)
CₙH₂ₙN+ (CH₃) ₃X⁻ (III)
where n is 16, 18, 20 or 22 and X⁻ is a chloride ion or methosulfate ion.

8. The composition as claimed in any of claims 1 to 7, comprising one or more cationic surfactants of the formula (IV)
[R¹CO-NH-CH₂-CH₂-CH₂-NR²R³R⁴]⁺ X⁻ (IV)
where R¹CO is a linear or branched, saturated or unsaturated acyl radical having 16 to 32 carbon atoms, R² and R³ are each methyl, R⁴ is H or methyl and X⁻ is an anion selected from chloride, methosulfate, lactate, citrate, glutamate and glycolate.

9. The composition as claimed in any of claims 1 to 8, wherein component c) comprises one or more fatty alcohols from the group of lauryl alcohol, stearyl alcohol, cetyl alcohol, Guerbet alcohol and behenyl alcohol.

10. A cosmetic, dermatological or pharmaceutical composition as claimed in any of claims 1 to 9.

11. The composition as claimed in claim 10 in the form of an emulsion.

12. A cosmetic composition as claimed in claim 10 or 11 in the form of a hair treatment composition.

13. The composition as claimed in any of claims 1 to 11 in the form of a cream or lotion.

14. The use of one or more N-methyl-N-acylglucamines as claimed in claim 1 or 2 for thickening emulsions, wherein the N-methyl-N-acylglucamine(s) a) is/are used together with at least one fatty alcohol b) as claimed in claim 1 or 9, and the ratio of components a:b is 1:9 to 5:5.

## Revendications

1. Composition contenant
a) une ou plusieurs N-méthyl-N-acylglucamines, au moins 50% en poids des N-méthyl-N-acylglucamines présentant un groupe C₁₆-acyle, C₁₇-acyle et/ou C₁₈-acyle,
b) un ou plusieurs alcools gras,
c) un ou plusieurs agents tensioactifs cationiques monomères,
d) le cas échéant d'autres additifs et
e) de l'eau.

2. Composition selon la revendication 1, au moins 50% en poids des N-méthyl-N-acylglucamines correspondant à des composés de formule (I), 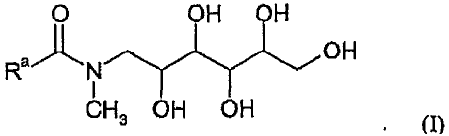 R^{a}-CO étant dérivé de l'acide palmitique, de l'acide stéarique, de l'acide oléique ou de l'acide linoléique.

3. Composition selon la revendication 1 ou 2, contenant
(a) 0,1 à 5% en poids du composant (a),
(b) 0,1 à 10% en poids du composant (b),
(c) 0,1 à 5% en poids du composant (c),
(d) 0 à 30% en poids du composant (d) et
(e) 99,8 à 50% en poids du composant (e),
la somme des composants (a), (b), (c), (d) et (e) valant 100% en poids.

4. Composition selon l'une quelconque des revendications 1 à 3, contenant
(a) 0,2 à 3% en poids du composant (a),
(b) 0,1 à 10% en poids du composant (c),
(c) 0,1 à 3% en poids du composant (b),
(d) 0 à 30% en poids du composant (d) et
(e) 99,6 à 54% en poids du composant (e),
la somme des composants (a), (b), (c), (d) et (e) valant 100% en poids.

5. Composition selon l'une quelconque des revendications 1 à 4, le composant a) contenant au moins 80% en poids de N-méthyl-N-acylglucamines, qui présentent un groupe C₁₆-C₁₇-acyle et/ou C₁₈-acyle.

6. Composition selon l'une quelconque des revendications 1 à 5, contenant un ou plusieurs agents tensioactifs cationiques de formule (II) 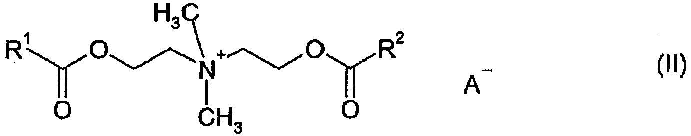 dans laquelle
R¹CO et R²CO représentent, indépendamment l'un de l'autre, des groupes acyle saturés, linéaires ou ramifiés comprenant 18 à 24 atomes de carbone et
A⁻ représente un contre-ion,
et la quantité totale de groupes C₁₈₋₂₃-alkyl-COO-, par rapport à tous les groupes R¹COO- et R²COO-, étant de 40,0% en poids ou plus.

7. Composition selon l'une quelconque des revendications 1 à 6, contenant un ou plusieurs agents tensioactifs cationiques de formule (III)
CₙH₂ₙN⁺(CH₃) ₃X⁻ (III)
dans laquelle n vaut 16, 18, 20 ou 22 et X- correspond à un ion de chlorure ou de méthosulfate.

8. Composition selon l'une quelconque des revendications 1 à 7, contenant un ou plusieurs agents tensioactifs cationiques de formule (IV)
[R¹CO-NH-CH₂-CH₂CH₂NR²R³R⁴] ⁺X⁻ (IV)
dans laquelle R¹CO représente un radical acyle linéaire ou ramifié, saturé ou insaturé comprenant 16 à 32 atomes de carbone, R² et R³ représentent méthyle, R⁴ représente H ou méthyle et X⁻ représente un anion, choisi parmi chlorure, méthosulfate, lactate, citrate, glutamate et glycolate.

9. Composition selon l'une quelconque des revendications 1 à 8, le composant c) contenant un ou plusieurs alcools gras du groupe constitué par l'alcool laurylique, l'alcool stéarylique, l'alcool cétylique, un alcool de Guerbet et l'alcool béhénylique.

10. Composition cosmétique, dermatologique ou pharmaceutique selon l'une quelconque des revendications 1 à 9.

11. Composition selon la revendication 10 sous forme d'une émulsion.

12. Composition cosmétique selon la revendication 10 ou 11 sous forme d'un agent de post-traitement des cheveux.

13. Composition selon l'une quelconque des revendications 1 à 11 sous forme d'une crème ou d'une lotion.

14. Utilisation d'une ou de plusieurs N-méthyl-N-acylglucamines selon la revendication 1 ou 2 pour épaissir des émulsions, la ou les N-méthyl-N-acylglucamines a) étant utilisées ensemble avec au moins un alcool gras b) selon la revendication 1 ou 9 et le rapport des composants a:b valant 1:9 à 5:5.
